# EUROPEAN PATENT APPLICATION

(11) **EP 2 518 138 A1**
(43) Date of publication of application: **31.10.2012**
(21) Application number: 11164378.9
(22) Date of filing: 29.04.2011
(51) Int. Cl.: C12N 1/20, C12N 11/04, C12N 11/08, B65D 1/02, B32B 27/18, B32B 27/36, B32B 33/00, C08J 3/20, C08J 5/00

(54) **Method for producing a polymer product from polar lipids from archaea as carriers of living microbial cells and biological barriers in plastic and textile**

(71) Applicant: Resilux, 9230 Wetteren (BE)
(72) Inventor: Dierickx, William, 9070 Destelbergen (BE); de Cuyper, Dirk, 9070 Destelbergen (BE); Poklar Ulrih, Natasa, 1000 Ljubljana (SI); Gunde-Cimerman, Nina, 1000 Ljubljana (SI)
(74) Representative: Petsis, Christos

(57) **Abstract**

The invention relates to a method for producing a polymer product that is remarkable in that it comprises the following steps: a polymer is selected in form of granules; a set of living organisms is selected from among cells or cell products with regard to liposomes or archeosomes as carriers of living microbial cells, such as extracellular layers or capsules, to be incorporated into liposomes or archaeosomes; granules are coated by thin layer culture with said living organisms; the polymer is moulded into said product, wherein aggregates are then formed inserting the mentioned cells into said liposomes or archeosomes, with formation of liposome-bioaggregate or archeosome-bio-aggregate. It also relates to a perform achieved with said method comprising a polymer containing living organisms.

## Description

### Field of the invention

This invention relates to polyether lipids, to planar, to unilamelar and multilamelar liposomes and the archaea comprising polyether lipids and their use as solute, gas, thermal and UV-barriers in plastic materials and textile, and to liposomes as carriers of living microbial cells and their use as solute, thermal and UV-barriers in plastic materials and textile.

### Prior Art

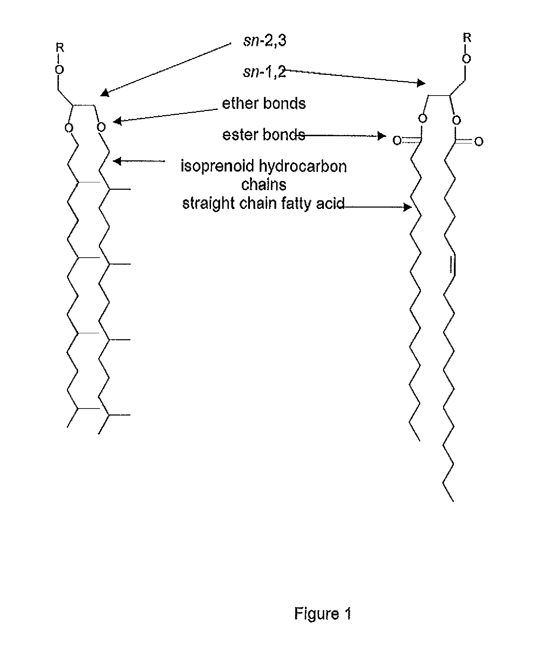

Polar archaeal lipids are generally composed of a core lipid and a phosphodiester bonded polar head groups or glycosides that are linked to one of the core lipids. Four characteristics unique to archaeal lipids compared with bacterial lipids have been identified (Figure 1) (Koga and Morii 2006): (i) Glycerophosphate backbone of archaeal lipids is composed of sn-glycerol-1-phosphate (G-1-P), which is an enantiomer of *sn-*glycerol-3-phosphate (G-3-P) in bacterial phospholipids. Hydrocarbon chains are though bound at the sn-2 and -3 positions of the glycerol moiety in archaeal lipids, whereas bacterial lipids have sn-1,2-radyl chains. (ii) Hydrocarbon chains are bonded to the glycerol moiety exclusively by ether linkages in archaeal polar lipids in contrast to bacterial lipids, most of which have ester linkages. (iii) The hydrocarbon chains of polar lipids in Archaea are highly methyl-branched isoprenoids and isopranoids. Bacterial counterparts are mostly straight chain fatty acids. (iv) Significant number of archaeal species contain bipolar lipids with a tetraether core that span through membranes (bolaform amphiphilic), which are rarely found in Bacteria.

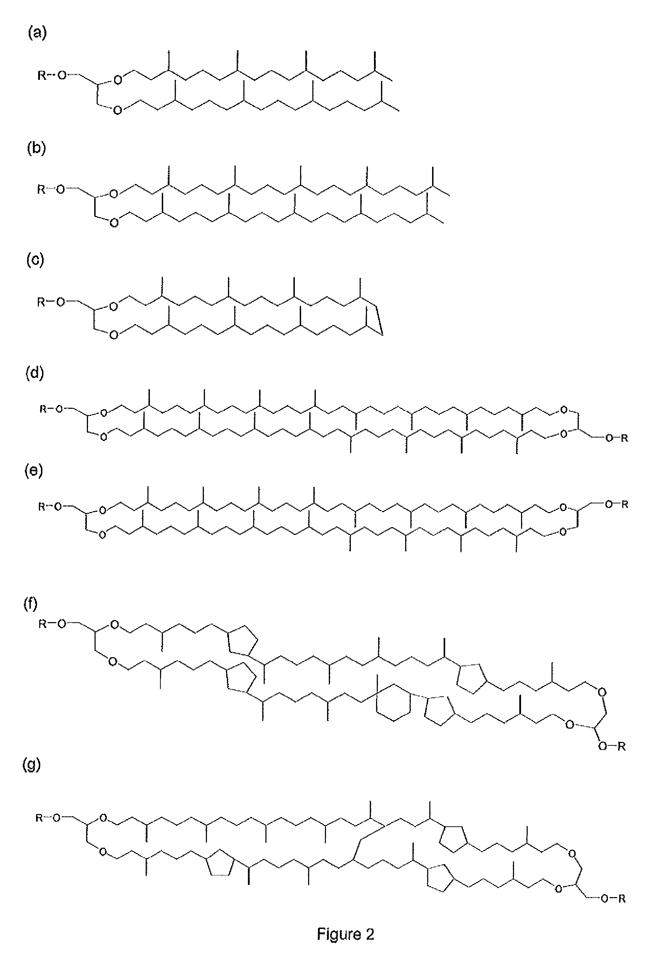

Basically, major core lipid structures consist of the ubiquitous standard archaeol (2,3-di-O-diphytanil-sn-glicerol) consisting mainly of C_{20,20} alkyl chains and/or the standard caldarchaeol (2,2',3,3'-tetra-O-dibiphytanil-sn-diglycerol) with C_{40,40} carbon chains (Figure 2). Variations to these standard lipid cores include archaeols with increased number of C₅ units, or caldarchaeols with cyclopentane rings within the C₄₀-phytanil chains (Figure 2) (Patel and Sprott 1999). Recently a numerous new core lipid structures have been elucidated (Koga and Morii 2005). Those include isocaldarchaeol from *Methanothermobacter marburgensis* (mixture of regioisomers with antiparallel and parallel arrangements of two glycerol moieties), H-shaped caldarchaeol from *Methanothermus fervidus* (Morii et al. 1998), C₄₀ isoprenoid hydrocarbons with cyclopentane rings at unusual positions isolated from sedimentary organic matter samples of water column from several marine environments (Hoefs et al. 1997), macrocyclic archaeol with one or two cyclopentane rings in the byphytanediyl chain (Stadnitskaia et al. 2002), alkyl ether type core lipids from *Methanopyrus kandleri* and *Methanococcoides burtonii* (Sprott et al. 1997a), and many more new lipids that are minor modifications of known lipids of archaea (Koga and Morii 2005).

The polar head groups of archaeal lipids are phospholipids or glycosides that are linked to one of the core lipids. The most common phospholipids are phosphoserin, phosphoinositol, phosphoglycerol, phosphoetanolamin and many phosphoglyicolipids, among them the most common carbohydrates found among archaeal lipids are glucose, gulose, mannose, galactose, inositol and *N*-acetylglucosamine, which can form mono-, di-, or oligosaccharides on one or both sides of caldarchaeol. Phosphoglycolipids with two polar head groups on both sides of the caldarchaeol may have glycerophosphate as the phosphoester moiety on one side and gulose alone or glucose and mannose, which form mono-, di-, or oligosaharides as the sugar moiety on the other side as is in the case of *Thermoplasma acidophilum* (Shimada et al. 2008). Replacement of one glycerol moiety of the core lipid backbone by a nonitol has also been observed (De Rosa and Gambacorta 1988). The occurrence of unusual carbohydrate β-D-galactofuranosyl units has been found in methanogens (Gambacorta et al. 1995), but it has not been found in thermoacidophiles (Tables 1 and 2) since the five-member rings in such environments are rapidly hydrolyzed.

With regard to Barrier for O₂, CO₂, H⁺ protons, small organic molecule, UV-light and heat, ether linkages are more stable than ester over a wide range of pH, and the branching methyl groups help to reduce both crystallization (membrane lipids in the liquid crystalline phase at ambient temperature) and membrane permeability (steric hindrance of the methyl side group). The saturated alkyl chains would impair stability towards oxidative degradation particularly in halophiles that are exposed to air and sunlight (Benvegnu et al. 2008). The membranes of methanogens and thermoacidophiles essentially consist of bipolar monolayer structures (Table 2). The high proportions of glycosylated lipids presented in membranes of thermoacidophiles and methanogenes may further stabilize their membrane structures through the interglycosyl headgroup hydrogen bonding. The presence of large sugar heads towards the convex surface of the membrane is likely to promote an asymmetric orientation, thus making the monolayer organization easier. Furthermore the flux of small molecules and protons through archaeal bipolar tetraether lipid membranes is considerably reduced as the result of the particular physical structure of the lipid monolayer. Finally, the presence of the cyclic diether structures in species isolated from deep-sea hydrothermal vents may be related to the high pressures under which these archaea live.

It has been observed, that archaeal cultures grown at different conditions exhibit different lipid membrane ratios as is in the case of *Archaeoglobus fulgidus* where change in tetraether-to-diether lipid ratio and number of pentacycles in the hydrocarbon chains was observed between cultures grown at 70°C or 89°C, respectively (Lai et al. 2008). The membrane of acidothermophiles is composed of 100% tetraether lipids, while for (neutro)-hyperthermophiles the ratios between tetraether and diether lipids is strain dependent.

*In vitro* study using the liposomes (archeosomes) made from caldarchaeol lipids of *Sulfolobus acidocaldarius,* which has an optimal growth temperature of 85°C at pH 2.0, at temperatures below 40°C no proton permeability was observed (Elferink et al. 1994). In the similar study of the major polar lipids from *S. acidocaldarius,* it was shown that membrane surface charge was responsible for low 5,6-carboxylfluorescence permeability, but not for proton permeability (Elferink et al. 1994). More examples are listed in following paragraphs describing archaeosomes.

It is known that the structural differences between lipids affect solute permeation through the membranes. In the polar headgroup regions of bipolar tetraether liposomes there is an extensive network of hydrogen bonds, which should generate a high electrical dipole potential, thus hindering solute permeability through membranes. Unlike non-archaeal monopolar diester/diether lipids, archaeal bipolar tetraether lipids do not have the hydrocarbon terminal methyl groups, instead the biphytanyl hydrocarbon chains are linked covalently from one polar end to the other, lacking the midplace spacing. Further, there are cyclopentane rings in the phytanyl chains, which provide additional rigidity to the membrane.

As to Artificial archaeal lipid membranes, in particular plannar lipids, more particularly liposomes or archaeosomes, archaeal lipids were shown to be an excellent source for the formation of liposomes (archaeosomes) with remarkable thermostability and tightness against solute leakage (Gambacorta et al. 1995). Ether lipids are also resistant to enzymatic degradation by phospholipases and archaeal liposomes are exceptionally stable, they do not fuse or aggregate during storage at 4°C over a period of four months (De Rosa 1996).

Membrane stability can be conveniently monitored by determining the release of fluorescent dyes originally trapped in the intravesicular compartment of the liposomes. In particular, several studies have focused on investigation of membranes made solely from bipolar lipid fractions. Different physicochemical properties (structure, dynamics, and polymorphism, thermal and mechanical stability) of bipolar lipid fractions extracted from several archaeal species have been investigated (Chang 1994; Elferink et al. 1994; Yamauchi et al. 1993).

Arakava and his coworkers (Arakawa et al. 1999) investigated the polymorphism and physicochemical properties of the macrocyclic lipids by synthetic 72-membered macrocyclic tetraether lipids. DSC, ³¹P NMR and electron microscopy analysis studied the physicochemical features of diphospholipids. The cyclic tetraetherlipids appeared to show lower phase transition temperature (T_{c}). Fluorescence studies have shown that the passive proton permeability in bipolar tetraether liposomes isolated from S. *acidocaldarius* is lower and less temperature sensitive than that in liposomes composed of monopolar diester lipids, although the permeability increases with temperature in all liposomes. It has been proposed that low proton permeability is due to the chemical structure of tetraether lipids and their monolayer organization, especially the cyclopentane rings and the network of hydrogen bonds between the sugar residues exposed at the outer face of the membrane (Chong et al. 2003, Mathai et al. 2001).

In general, it was shown that at a given temperature, the bolaform lipid chains are more ordered and less flexible than in conventional bilayer membranes. Only at elevated temperatures (80°C) does the flexibility of the chain environment in tetraether lipid assemblies approach that of fluid bilayer membranes (Bartucci et al. 2005). By examination of water, solute (urea and glycerol), proton and ammonia permeability of archaeosome it was shown that macrocyclic archaeol and caldarchaeol lipids reduced the water, ammonia, urea and glycerol permeability significantly (6-120-fold) compared to dipalmitoylphosphatidylcholine (DPPC) liposomes (Mathai et al. 2001).

The presence of an ether bond reduced the apparent proton permeability for 3-fold compared to DPPC liposomes. The presence of the ether bond and phytanyl chains did not significantly affect these permeabilities. The presence of macrocycllic archaeol and caldarchaeol structure further reduced apparent proton permeability by 10-17 fold (Mathai et a. 2001). Komatsu and Chong have suggested that the low leakage rates of anionic probe at neutral pH are due to the negative charges of polar tetraether lipid membrane surface in addition to the tight and rigid lipid packing (Komatsu and Chong 1998). Some studies have shown that the leakage rate of entrapped dyes increases with decreasing the content of tetraether lipids and increases in the presence of fusogenic agents such as calcium ions and polyethylene glycol (PEG). These observations and many others suggest that the degree of hydration and conformation of the lipid polar headgroup are crucial for the tetraether membrane integrity, Furthermore, the release of entrapped dyes in bipolar tetraether liposomes is less temperature sensitive than that in non-archaeal monopolar diester liposomes (Mathai et al. 2001), suggesting a low thermal expansibility for tetraether liposomes (Chong et al. 2003). This general property is important for thermophiles, since they may experience temperature fluctuations in their native environment.

### Summary of the invention

There is proposed according to the invention a method for producing a polymer product, that is remarkable in that it comprises the steps of selecting a polymer in form of granules, further selecting a set of living organisms from among cells and/or cell products with regard to liposomes or archeosomes as carriers of living microbial cells, in particular such as extracellular layers or capsules, to be incorporated into liposomes or archeosomes, further coating granules by thin layer culture with said living organisms, still further moulding the polymer into said product, wherein aggregates are then formed inserting the mentioned cells into the mentioned liposomes or archeosomes, resulting in the formation of a liposome-bioaggregate or a archeosome-bio-aggregate. The method proposed is in this order or in a different one as well.

With regard to liposomes or archeosomes as yet undescribed carriers of living microbial cells, according to the invention, a set of organisms is presently selected from among cells and/or cell products, which are thus incorporated into liposomes or archeosomes, finally resulting in the formation of what is known as liposome-bioaggregate or a archeosome-bio-aggregate.

According to a particular embodiment of the invention, cells can be selected from a category known as prokaryotic or eukaryotic vegetative cells and/or a phase of inactive or dormant stage such as sexual or asexual spores or cysts or meristematic clumps.

According to a more particular embodiment of the invention, said cells, mersistematic clumps or cell dormant stages, encapsulated in liposomes or archeosmoes should withstand extremely dry conditions and temperatures well above 100° C and act as permanent oxygen or CO₂ barriers, UV blockers and potentially colorings.

According to an advantageous embodiment of the invention, a reliable, slow and prolonged diffusion of organic molecules can be achieved through said polymers into said liposomes or archeosomes and to the encapsulated microbial cells, being realized, creating in the polymer a moist and fluctuating environment, that could activate slow metabolic microbial processes.

Depending on the selected microbial cell type, its metabolic activity within the archeosome/liposome included in the polymer, the composition of the selected archeosome or liposome as carrier, the selected archeosome or liposome could or could not be degraded, enabling enlargement of immediate space within the polymer for the selected encapsulated microbial cell, enabling its slight expansion or growth and basic nutrition, creating an active and/or passive barrier for oygen ad other gases permeation.

According to a further embodiment of the method of the invention, the bio component of the archeosome liposome - bioagreggates could be a type of yeast with a dry spore, such as for example *Saccharomyces cerevisiae,* which would be able to withstand the physicochemical conditions required for the inclusion of liposome archeosome-bio-aggregates into the polymer.

Another type of yeast could be pleomorphic yeasts such as *Hortaea werneckii,* able to grow as yeast cells, hyphal cells, budding hyphae, spores or meristematic clumps. Another type of fungal cells are molds such as *Aspergillus spp.* and their conidia, sexual spores, hyphal fragments, mycelial strands and dormant structures such as chlamidial cells or sclerotia.

Instead of fungal cells algal cells, could be incorporated into the archeosome liposome - bioagreggates, such as Haematococcus or Dunaliella salina, or their spores.

The bio component could be also a mixture of algal and fungal cells.

The bio component of the archeosome liposome - bioagreggates could be prokaryotic cells such as a type of bacterial cell with a dry spore or endospore, like for example *Bacillus spp.* or lactic acid bacteria or an archeal cell such as halophilic Halococcus or thermophilic Aeropyrum pernix.
According to a specific embodiment of the method according to the invention, said living organisms are selected from among the extremophiles.

According to a more specific embodiment of the invention, said living organisms are selected from among the species Bacillus Subtilis, in particular the one bearing No. ID9698.

According to a still more specific embodiment of the invention, said polymers are selected from among the family of the thermoplastic polymers, in particular from among the family of the polyolefins, or polyesters, more particularly from among the family of the polyethylenes, or polypropylenes, even more particularly polyethylene terephthalate (PET), yet more particularly in the form of PET granules that are coated with living organisms brought to a temperature higher than 260°C during the injection molding process.

According to an alternative embodiment of the invention, said polymer is made of PETG, with a lower melting temperature than standard PET.

According to a useful embodiment of the invention, said product is a preform intended to be further processed to a container, in particular wherein said preform consists in at least three layers, an intermediate layer made of PETG between two outer layers, more particularly wherein said outer layers are made of PET, even more particularly wherein the injection molding is a co-injection molding where the PETG is injected colder than the layers on both sides of it, even more particularly wherein the PETG granules are coated with living organisms, and in wherein it is brought to a temperature higher than 200°C during the injection molding process.

According to a further alternative embodiment of the invention, said product is a polymer film.

According to an additional embodiment of the invention, said living organisms are introduced in the polymer in a liquid stage during injection molding, at a temperature above 260°C.

According to a further additional embodiment of the preform according to the invention, it is intended for producing containers made of a polymer containing living organisms, consisting of at least one layer, wherein said layer is made of a polyethylene terephthalate glycol containing living organisms, in particular wherein said preform consists in at least three layers, the layer made of PETG being an intermediate layer between the two other layers, more particularly wherein said polymer product comprises a layer of a preform intended to make a container, said layer being a barrier layer, preferably a gas barrier layer, more preferably an oxygen barrier layer, more particularly wherein said living organisms in the PETG belong to the species Bacillus Subtilis, yet more particularly wherein said living organisms are introduced in the PETG in a liquid stage during injection moulding, at a temperature above 200°C.

According to a still further embodiment of the invention, said polymer contains at least two different types of living organisms.

Finally, the present invention also relates to a container made from a preform as defined in any of the appended sub-claims directed thereto, in particular wherein said living organisms in said container are active in a temperature range reaching at least 4 to 30°C once the container has been filled, more particularly wherein at least two different types of living organisms are selected for coating granules.

Further details are illustrated in the following examples with reference to the attached drawings, which are described below.

### Brief description of the drawings

Figure 1 shows typical structures of archaeal phospholipid compared with bacterial phospholipid. Four differences are shown. -R stands for polar head group.
Figure 2 shows typical archaeal polar lipid cores. (a) archaeol (C20,20), (b) archaeol (C25,25), (c) macrocyclic arhaeol, (d) caldarchaeol, (e) isocaldarchaeol, (f) crenarchaeol, (g) H-shaped caldarchaeol containing three cyclopentane rings.

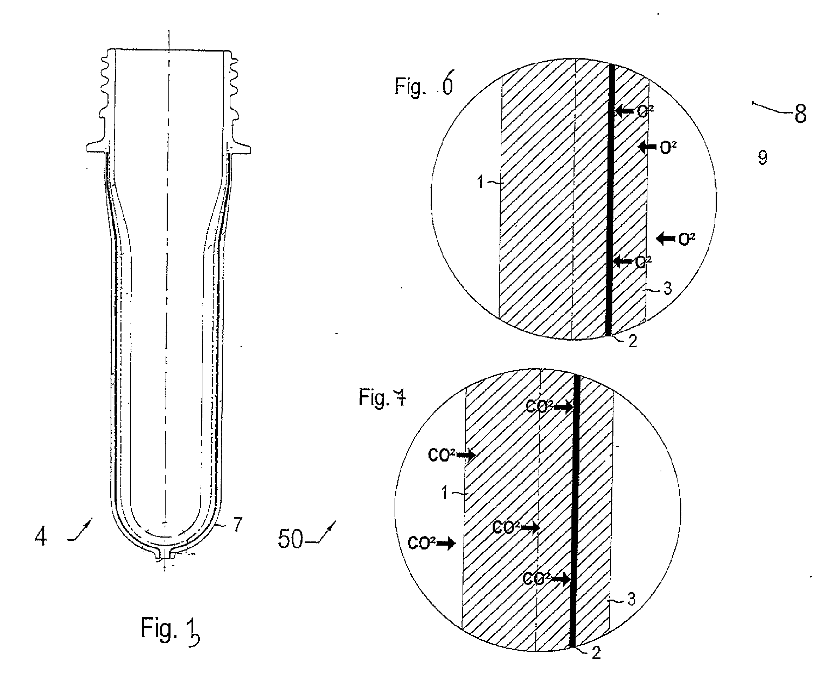

Fig. 3 is a diagrammatic representation of a cross section in a central plane of an embodiment of a preform according to the invention.

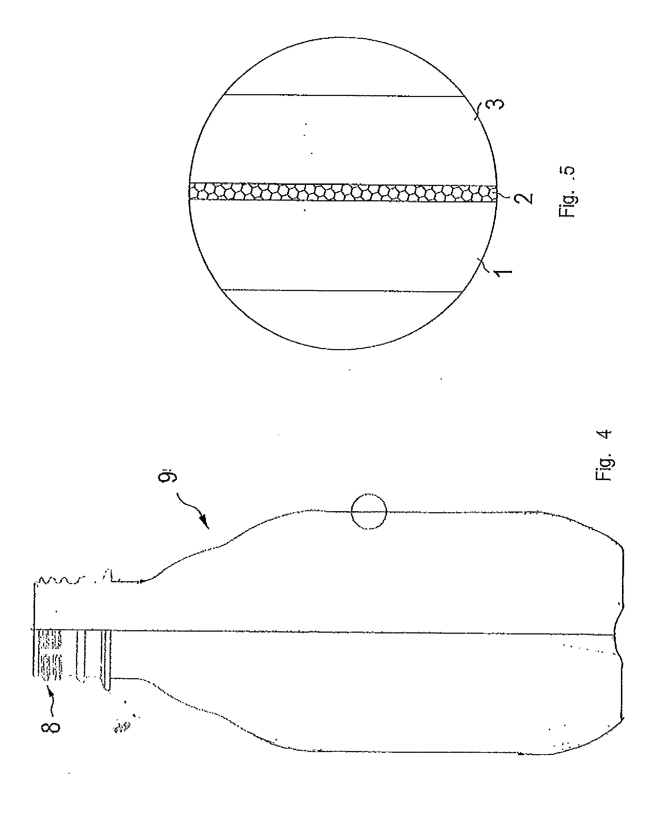

Fig. 4 is a mixed representation of a container according to the invention partially in a front view and partially in a cross section.
Fig. 5 is an enlarged view of a detail of the wall of the container represented in Fig. 4 according to the invention.
Fig. 6 & 7 are each a diagrammatic representation of the functional working of an essential part of said container according to the invention, with an enlarged view of a detail of the wall thereof.

### Description

### EXAMPLE

Regarding the isolation, purification of lipids and vesicles preparation, the polar lipid methanol fraction (PLMF) can be purified from the lyophilized archaea cells. Lipids are fractionated with adsorption chromatography on a Sep-Pak® Vac 20cc Silica cartridges (Waters) using following elution sequence: chloroform (170 mL); acetone-methanol (9/1) (250 mL); methanol (170 mL). Fractions can be analyzed by TLC with solvent of chloroform/methanol/acetic acid/water (85/30/15/5). The methanol fraction containing polar lipids is used for further analysis. The methanol lipid solution is dried by slow evaporation under a constant flow of dry nitrogen. Following the removal of the last traces of organic solvent in a vacuum, mass of lipids in 10 mL round-bottom glass flask is determined by subtracting the mass of the same clean flask. Lipids are then hydrated in different aqueous buffer solutions. Multilamelar vesicles (MLV) are prepared by vortexing the lipid suspension for 10 min. MLV are further transformed to small unilamelar vesicles (SUV) by 30 min sonication with 10 s on-off cycles at 50 % amplitude with a Vibracell Ultrasonic Disintegrator VCX 750 of Sonics and Materials, Newtown, USA.

### EXAMPLE:

Result of using the archaeal cells as a passive barrier for 02 are set out hereafter. Some new applications are set out below.

Based on the literature data of physicochemical properties of polyether lipids from archaea, the abovementioned poly ether lipids, with or without included microbial cells or spores, can be used in the production of plastic materials and textile to achieve considering the following:
- Lower permeability for hydrogen protons. In textile industry this means protections against acidic environment (lab coat, glows, ...)
- Lower and controlled permeability for O₂, CO₂ (in production of plastic bottles for soft drinks, fruits juice, olive and other oil sensitive to oxidation processes, ...)
- Lower permeability for smaller organic molecule (water filters for removing organic compounds
- Lower permeability for water (water proofed textile, ...)
- Protection against heat and UV-light (textile, plastic,...
- Coloring with biological pigments, included in the cell wall or membrane of the microbial cells. Additionally coloring prevents oxidation of products in the bottle, due to scavenging abilities of pigments.

Up to date applications of living cells incorporated in liposomes or archeosomes are presently proposed according to this invention, including up to date applications of poly ether lipids.

Both *in vitro* and *in vivo* studies indicate that archaeosomes are safe. Their pH and thermal stability, tissue distribution profiles, as well as the adjuvant activity of archaeosome formulations, indicate that they may offer a superior alternative for several biotechnological applications, including delivery systems for drugs, genes or cancer imaging agents (Table 3).

As an ideal vector in drug and gene delivery should be highly efficient in delivering the drug in a target-specific manner (various molecules can be incorporated into archaeosomes, or associated with the vesicles to target specific tissues), stable *in vitro* as well as *in vivo,* nontoxic and non-immunogenic, archaeosomes delivery systems answer most of these conditions. The two important observations are: (i) archaeosomes promote strong humoral and cell-mediated (Th1/Th2) immune reactions to soluble-entrapped proteins. More importantly, archaeosomes induce CD8+ cytotoxic T lymphocyte responses (critical for protection against cancer and intracellular infections) to entrapped proteins. Thus, archaeosomes are self-adjuvanting delivery vesicles and (ii) better immunization with archaeosomes is achieved. Recently, two excellent reviews were published covering the archaeosome adjuvants from immunological capabilities to mechanism(s) of action and a synthetic route for the preparation of symmetrical and unsymmetrical archaeal tetraether-like analogues have been described for use in these purposes. Archaeal lipids have also been proposed as monomers for bioelectronics for several reasons, the most important being that their bipolar tetraether structure offers novel opportunities for protein-lipid interactions, which are of interest for the assembly of electronic devices based on redox proteins or enzymes. Some other applications include use of archaeal lipids as taxonomic markers and as novel lubricants for engines since they are chemically stable at high temperatures, lubricate, either with or without a carrier lubricant and provide a friction coefficient of less than 0,1.

**Table 1: Biotechnological applications of tetraether archaeal lipids**

| **Application** | **Reference** |
|---|---|
| Taxonomic markers | (Gambacorta et al. 1995; Koga and Morii 2005) |
| Lubrication | (Chang 1992) |
| Preparation of membranes for separation processes | (Bauer et al. 1983) |
| Antigen delivery systems | (Patel and Sprott 1999) |
| Protein delivery system | (Sprott et al. 1997b, 1999) |
| | |
| Immobilization of the molecule | (Berzina et al. 1995;Berzina et al. 1997) |
| lmunization/vaccine adjuvant | (Sprott et al. 2003; Krishnan and Sprott 2008) |
| Food industry- | (Mozafari et al. 2006) |
| Bioelectronics | (De Rosa et al. 1994) |
| Ultratinlayers for biosensors | (Meister and Blume 2007) |
| Biomimetric materials (film deposition on solid surface application in fitter permeability) | (Muller et al. 2006) |
| Synthetic preparation of archaeal lipid ana analogues | (Brard et al. 2007) |

### References

Arakawa K, Kano H, Eguchi T, Nishiyama Y, Kakinuma K (1999) Significance of the 72-membered macrocyclic structure found in archaeal membrane lipids: Model studies of the macrocyclic tetraether diphospholipids by calorimetric, P-31 NMR, and electron microscopic analyses. Bull Chem Soc Jpn 727:1575-1581.
Bartucci R, Gambacorta A, Gliozzi A, Marsh D, Sportelli L (2005) Bipolar tetraether lipids: Chain flexibility and membrane polarity gradients from spin-label electron spin resonance. Biochemistry 4445:15017-15023.
Bauer S, Heckmann K, Six L, Strobl C, Blöcher D, Henkel B, Garbe T, Ring K (1983) Hyperfiltration through crosslinked monolayers II. Desalination 481-3:369-378.
Benvegnu T, Lemiegre L, Cammas-Marion S (2008) Archaeal lipids: Innovative materials for biotechnological applications. Eur J Org Chem 28:4725-4744.
Berzina TS, Troitsky VI, Vakula S, Riccio A, Morana A, De Rosa M, Dante S, Maccioni E, Rustichelli F, Accossato P, Nicolini C (1995) Langmuir-Blodgett-Films Of Bipolar Lipids From Thermophilic Archaea. Mat Sci Eng C-Biomim 31:13-21.
Berzina TS, Troitsky VI, Vakula S, Riccio A, De Rosa M, Nicolini C (1997) Surface potential study of selective interaction of potassium ions with valinomycin in Langmuir-Blodgett films. Mat Sci Eng C-Biomim 51:1-6.
Brard M, Laine C, Rethore G, Laurent I, Neveu C, Lemiegre L, Benvegnu T (2007) Synthesis of archaeal bipolar lipid analogues: A way to versatile drug/gene delivery systems. J Org Chem 7222:8267-8279.
Chang EL (1992). New class of lubricants derived from archaebacterial lipids. The United States of America as represented by the Secretary of the Navy. United States Patent 5098588
Chang EL (1994) Unusual thermal stability of liposomes made from bipolar tetraether lipids. Biochem Biophys Res Commun 2022:673-679.
De Rosa M, Gambacorta A, Trincone A, Basso A, Zillig W, Holz I (1987) Lipids of Thermococcus celer, A sulfur-reducing archaebacterium - Structure and biosynthesis. Syst Appl Microbiol 91-2:1-5.
De Rosa M, Gambacorta A (1988) The lipids of archaebacteria. Prog Lipid Res 273:153-175.
De Rosa M, Morana A, Riccio A, Gambacorta A, Trincone A, Incani O (1994) Lipids of the Archaea: a new tool for bioelectronics. Biosens Bioelectron 99-10:669-675.
De Rosa M (1996) Archaeal lipids: structural features and supramolecular organization. Thin Solid Films 284-285:13-17.
Elferink MGL, de Wit JG, Driessen AJM, Konings WN (1994) Stability and proton-permeability of liposomes composed of archaeal tetraether lipids. BBA-Biomembranes 11932:247-254.
Gambacorta A, Gliozzi A, De Rosa M (1995) Archaeal lipids and their biotechnological applications. World J Microb Biot 111:115-131.
Hoefs MJL, Schouten S, De Leeuw JW, King LL, Wakeham SG, Damste JSS (1997) Ether lipids of planktonic Archaea in the marine water column. Appl Environ Microbiol 638:3090-3095.
Koga Y, Morii H (2006) Special methods for the analysis of ether lipid structure and metabolism in Archaea. Anal Biochem 3481:1-14.
Koga Y, Morii H (2007) Biosynthesis of ether-type polar lipids in Archaea and evolutionary considerations. Microbiol Mol Biol Rev 711:97-120.
Komatsu H, Chong PLG (1998) Low permeability of liposomal membranes composed of bipolar tetraether lipids from thermoacidophilic archaebacterium Sulfolobus acidocaldarius. Biochemistry 371:107-115.
Krishnan L, Sprott GD (2008) Archaeosome adjuvants: Immunological capabilities and mechanism(s) of action. Vaccine 2617:2043-2055.
Lai D, Springstead J, Monbouquette H (2008) Effect of growth temperature on ether lipid biochemistry in Archaeoglobus fulgidus. Extremophiles 122:271-278.
Mathai JC, Sprott GD, Zeidel ML (2001) Molecular mechanisms of water and solute transport across archaebacterial lipid membranes. J Biol Chem 27629:27266-27271.
Meister A, Blume A (2007) Self-assembly of bipolar amphiphiles. Curr Opin Colloid In 123:138-147.
Morii H, Eguchi T, Nishihara M, Kakinuma K, König H, Koga Y (1998) A novel ether core lipid with H-shaped C80-isoprenoid hydrocarbon chain from the hyperthermophilic methanogen Methanothermus fervidus. BBA-Lipid Lipid Met 13903:339-345.
Morii H, Yagi H, Akutsu H, Nomura N, Sako Y, Koga Y (1999) A novel phosphoglycolipid archaetidyl(glucosyl)inositol with two sesterterpanyl chains from the aerobic hyperthermophilic archaeon Aeropyrum pernix K1. BBA-Mol Cell Biol L 14363:426-436.
Mozafari MR, Flanagan J, Matia-Merino L, Awati A, Omri A, Suntres ZE, Singh H (2006) Recent trends in the lipid-based nanoencapsulation of antioxidants and their role in foods. J Sci Food Agr 8613:2038-2048.
Muller S, Pfannmuller M, Teuscher N, Heilmann A, Rothe U (2006) New method for surface modification of nanoporous aluminum oxide membranes using tetraether lipid. J Biomed Nanotechnol 21:16-22.
Poklar Ulrih N, Gmajner D, Raspor D (2009) Structural and physicochemical properties of polar lipids from thermophilic archaea. Appl Microbiol Biotechnol 84: 249-260. Patel GB, Sprott GD (1999) Archaeobacterial ether lipid liposomes (archaeosomes) as novel vaccine and drug delivery systems. Crit Rev Biotechnol 194:317 - 357.
Shimada H, Nemoto N, Shida Y, Oshima T, Yamagishi A (2008) Effects of pH and temperature on the composition of polar lipids in Thermoplasma acidophilum HO-62. J Bacteriol 19015:5404-5411.
Sprott GD, Agnew BJ, Patel GB (1997a) Structural features of ether lipids in the archaeobacterial thermophiles Pyrococcus furiosus, Methanopyrus kandleri, Methanothermus fervidus, and Sulfolobus acidocaldarius. Can J Microbiol 435:467-476.
Sprott GD, Tolson DL, Patel GB (1997b) Archaeosomes as novel antigen delivery systems. FEMS Microbiol Lett 1541:17-22.
Sprott GD, Brisson JR, Dicaire CJ, Pelletier AK, Deschatelets LA, Krishnan L, Patel GB (1999) A structural comparison of the total polar lipids from the human archaea Methanobrevibacter smithii and Methanosphaera stadtmanae and its relevance to the adjuvant activities of their liposomes. BBA-Mol Cell Biol L 14402-3:275-288.
Sprott GD Sad S, Fleming LP, Dicaire CJ, Patel GB, Krishnan L (2003) Archaeosomes varying in lipid composition differ in receptor-mediated endocytosis and differentially adjuvant immune responses to entrapped antigen. Archaea 13:151-64.
Stadnitskaia A, Baas M, Ivanov MK, Tjeerd CE, Weering V, Damste JSS (2002) Novel archaeal macrocyclic diether core membrane lipids in a methane-derived carbonate crust from a mud volcano in the Sorokin Trough, NE Black Sea. Archaea 1:165-173.
Yamauchi K, Doi K, Yoshida Y, Kinoshita M (1993) Archaebacterial lipids: highly proton-impermeable membranes from 1,2-diphytanyl-sn-glycero-3-phosphocoline. BBA-Biomembranes 11462:178-182.

## Claims

1. Method for producing a polymer product, **characterized in that** it comprises the following steps:
- a polymer is selected in form of granules;
- a set of living organisms is selected from among cells and/or cell products with regard to liposomes or archeosomes as carriers of living microbial cells, in particular such as extracellular layers or capsules, to be incorporated into liposomes or archaeosomes;
- granules are coated by thin layer culture with said living organisms;
- the polymer is moulded into said product,
- wherein aggregates are then formed inserting the mentioned cells into the mentioned liposomes or archeosomes, resulting in the formation of a liposome-bioaggregate or a archeosome-bio-aggregate.

2. Method according to the preceding claim, **characterized in that** said cells are selected from a category of prokaryotic or eukaryotic vegetative cells and/or a phase of inactive or dormant stage, such as sexual or asexual spores, or cysts or meristematic clumps.

3. Method according to the preceding claim, **characterized in that** said cells, mersistematic clumps or cell dormant stages, which are encapsulated in liposomes or archeosmoes, are selected to withstand extremely dry conditions and temperatures well above 100°C and act as permanent oxygen or CO₂ barriers, UV blockers and/or potentially colorings.

4. Method according to one of the preceding claims, **characterized in that** a slow and prolonged diffusion of organic molecules is realized through said polymers into liposomes or archeosomes and to the encapsulated microbial cells, thereby creating in the polymer a moist and fluctuating environment, that activates slow metabolic microbial processes.

5. Method according to one of the preceding claims, **characterized in that** an active and/or passive barrier for oxygen and other gases permeation is created by enlargement of immediate space within the polymer for the selected encapsulated microbial cell, enabling its slight expansion or growth and basic nutrition, enabled by degradation or not of the selected archeosome or liposome, depending on the selected microbial cell type, its metabolic activity within the archeosome/liposome included in the polymer, the composition of the selected archeosome or liposome as carrier.

6. Method according to one of the preceding claims, **characterized in that** the bio component of the archeosome liposome - bioagreggates is a type of yeast with a dry spore, in particular such as *Saccharomyces cerevisiae,* which is able to withstand the physicochemical conditions required for the inclusion of liposome archeosome-bio-aggregates into the polymer.

7. Method according to claim 5 or 6, **characterized in that** another type of yeast is pleomorphic yeasts, in particular such as *Hortaea werneckil,* able to grow as yeast cells, hyphal cells, budding hyphae, spores or meristematic clumps.

8. Method according to one of the preceding claims, **characterized in that** another type of cells are fungal cells molds, in particular such as *Aspergillus spp.* and their conidia, sexual spores, hyphal fragments, mycelial strands and dormant structures, in particular such as Chlamydial cells or sclerotia.

9. Method according to the preceding claim, **characterized in that** instead of fungal cells, algal cells are incorporated into the archeosome liposome - bioagreggates, in particular such as Haematococcus or Dunaliella salina, or their spores.

10. Method according to one of the preceding claims, **characterized in that** the bio component is a mixture of algal and fungal cells.

11. Method according to one of the claims 1 to 9, **characterized in that** the bio component of the archeosome liposome - bioagreggates is prokaryotic cells such as a type of bacterial cell with a dry spore or endospore, like *Bacillus spp.* or lactic acid bacteria or an archeal cell in particular such as halophilic Halococcus or thermophilic Aeropyrum pernix.

12. Method according to any one of the preceding claims, **characterized in that** said living organisms are selected from among the extremophiles.

13. Method according to the preceding claim, **characterized in that** said living organisms are selected from among the species Bacillus Subtilis, in particular the one bearing No. ID9698.

14. Method according to one of the preceding claims, in particular one of both preceding ones, **characterized in that** the polymers are selected from among the family of the thermoplastic polymers.

15. Method according to the preceding claim, **characterized in that** the polymers are selected from among the family of the polyolefins, or polyesters.

16. Method according to the preceding claim, wherein the polymers are selected from among the family of the polyethylenes, or polypropylenes.

17. Method according to the preceding claim, **characterized in that** said polymer is selected from polyethylene terephthalate (PET).

18. Method according to the preceding claim, **characterized in that** the PET granules coated with living organisms are brought to a temperature higher than 260°C during the injection molding process.

19. Method according to claim 14, **characterized in that** said polymer is made of PETG, with a lower melting temperature than standard PET.

20. Method according to any one of the preceding claims, in particular one of the claims 12 to 19, **characterized in that** said product is a preform (4) intended to be further processed to a container (9).

21. Method according to the preceding claim, **characterized in that** said preform (4) consists in at least three layers (1, 2, 3), in particular an intermediate layer (2), more particularly made of PETG, between two outer layers (1, 3).

22. Method according to one of both preceding claims, **characterized in that** said outer layers (1, 3) are made of PET.

23. Method according to the preceding claim, **characterized in that** the injection molding is a co-injection molding where the PETG is injected colder than the layers (1, 3) on both sides of it (2).

24. Method according to the preceding claim, **characterized in that** the PETG material is fed from granules that are coated with living organisms, and **in that** it is brought to a temperature higher than 200°C during the injection molding process.

25. Method according to any one of the preceding claims, in particular one of claims 12 to 19, **characterized in that** said product is a polymer film.

26. Preform for being processed to containers, **characterized in that** it is made with a method according to any one of the claims 1 to 24, thereby comprising a polymer containing living organisms.

27. Preform according to the preceding claim, when it depends on one of the claims 13 to 23, **characterized in that** said living organisms are introduced in the polymer in a liquid stage during injection molding, at a temperature above 260°C.

28. Preform according to the preceding claim, intended for producing containers made of a polymer containing living organisms, consisting of at least one layer (2), **characterized in that** said layer (2) is made of a polyethylene terephthalate glycol containing living organisms.

29. Preform according to the preceding claim, **characterized in that** said preform (4) consists in at least three layers (1, 2, 3), the layer made of PETG being an intermediate layer (2) between the two other layers (1, 3).

30. Preform according to the preceding claim, **characterized in that** said polymer product comprises a layer of a preform intended to make a container, said layer being a barrier layer, preferably a gas barrier layer, more preferably an oxygen barrier layer.

31. Preform according to the preceding claim, **characterized in that** said living organisms in the PETG belong to the species Bacillus Subtilis.

32. Preform according to the preceding claim, **characterized in that** said living organisms are introduced in the PETG in a liquid stage during injection moulding, at a temperature above 200°C.

33. Preform according to one of the preceding claims, **characterized in that** said polymer contains at least two different types of living organisms.

34. Container made from a preform as defined in any of the claims 26 to 33.

35. Container according to the preceding claim, **characterized in that** said living organisms are active in a temperature range reaching at least 4 to 30°C once the container (9) has been filled.

36. Container according to the preceding claim, **characterized in that** at least two different types of living organisms are selected for coating granules.
